# FASCICULE DE BREVET EUROPEEN

(11) **EP 1 374 885 B1**
(45) Date de publication et mention de la délivrance du brevet: **16.05.2007**
(21) Numéro de dépôt: 03370025.3
(22) Date de dépôt: 24.06.2003
(51) Int. Cl.: A61K 38/05, A61K 38/06, A61K 38/07, A61K 38/08, A23L 1/305

(54) **Utilisation d'au moins un peptide de la caséine "alphaS2" à activité inhibitrice de l'ACE pour la préparation de médicaments et d'aliments**
Verwendung von mindestens einem Peptid des alpha-S2 Caseins mit ACE hemmender Wirkung zur Vorbereitung von Arzneimitteln und Nahrungsmitteln
Use of at least one peptide of alpha-s2 casein with inhibiting activity of ACE for the preparation of medicaments and foodstuffs

(30) Priorité: 27.06.2002 FR 0208036
(43) Date de publication de la demande: 02.01.2004
(73) Titulaire: Ingredia, 62000 Arras (FR)
(72) Inventeur: Tauzin, Jerome, 59221 Bauvin (FR); Miclo, Laurent, 54600 Villers les Nancy (FR); Lefranc, Catherine, 59650 Villeneuve D'Ascq (FR); Boudier, Jean-François, 62217 Agny (FR); Gaillard, Jean-Luc, 14530 Luc sur Mer (FR)
(74) Mandataire: Hennion, Jean-Claude

(56) Documents cités:
- EP-A- 0 445 606
- FR-A- 2 650 955
- TAUZIN JEROME ET AL: "Angiotensin-I-converting enzyme inhibitory peptides from tryptic hydrolysate of bovine alphaS2-casein." FEBS LETTERS, (2002 NOV 6) 531 (2) 369-74. , XP004391663
- YAMAMOTO ET AL: "Antihypertensive effect of the peptides derived from casein by an extracellular proteinase from Lactobacillus helveticus CP790" JOURNAL OF DAIRY SCIENCE, AMERICAN DAIRY SCIENCE ASSOCIATION. CHAMPAIGN, ILLINOIS, US, vol. 77, no. 4, 1994, pages 917-922, XP002095595 ISSN: 0022-0302
- YAMAMOTO N ET AL: "PURIFICATION AND CHARACTERIZATION OF AN ANTIHYPERTENSIVE PEPTIDE FROM A YOGURT-LIKE PRODUCT FERMENTED BY LACTOBACILLUS HELVETICUS CPN4" JOURNAL OF DAIRY SCIENCE, AMERICAN DAIRY SCIENCE ASSOCIATION. CHAMPAIGN, ILLINOIS, US, vol. 82, no. 7, juillet 1999 (1999-07), pages 1388-1393, XP000850204 ISSN: 0022-0302

## Description

La présente invention concerne l'utilisation d'un ou plusieurs peptides de la caséine α_{S2} bovine, ayant une activité inhibitrice de l'enzyme de conversion de l'angiotensine I pour la préparation de médicaments, d'aliments et de compléments alimentaires à activité de type anti-hypertensive.

La caséine entière est un ensemble de protéines du lait qui a été largement étudié, par exemple par GROSCLAUDE (1), SWAISGOOD (2) et GRAPPIN et RIBADEAU-DUMAS (3). La chromatographie sur DEAE-cellulose permet de fractionner à partir de la caséine entière, les caséines γ, κ, β, α_{S1} et α_{S2}. Les séquences en acides aminés de ces caséines sont bien connues [EIGEL *et al.* (4), HOLT and SAWYER (5)]; en particulier, celle de la caséine α_{S2} a été déterminée par BRIGNON *et al.* (6) et STEWART *et al.* (7).

On sait déjà que certains fragments peptidiques de ces différentes caséines ont des activités biologiques diverses [CLARE and SWAISGOOD (8), MEISEL (9)]. En ce qui concerne la caséine α_{S2}, les peptides CNα_{S2}-(f165-203) [ZUCHT *et al*. (10)], CNα_{S2}-(f183-207) et CNα_{S2}-(f164-179) [RECIO and VISSER (11)] présentent une activité antibactérienne et les peptides CNα_{S2}-(f189-193), CNα_{S2}-(f190-197) et CNα_{S2}-(f198-202) inhibent l'enzyme de conversion de l'angiotensine I [CORVOL *et al.* (12)] avec des valeurs d'IC₅₀, qui est la quantité de peptide nécessaire pour inhiber 50% de l'activité enzymatique, égales à 580, 300 et 400 µM respectivement [MAENO *et al.* (13)]. Toutefois ces peptides ne présentent pas d'effet antihypertensif significatif *in vivo* sur des lignées de rats spontanément hypertendus 6 heures après l'administration orale d'une dose de 1 mg de peptide de synthèse/kg de rat [MAENO *et al.* (13)].

L'enzyme de conversion de l'angiotensine I, dénommée ci-après l'ECA joue *in vivo* un rôle clé dans la régulation de la pression artérielle [WEBER (14)]. Les inhibiteurs de l'ECA (captopril, benazepril, enalapril, lisinopril...) [PIEPHO (15)] sont une des principales classes de molécules utilisées pour lutter contre l'hypertension. Ils sont particulièrement indiqués pour les patients diabétiques et les insuffisants cardiaques ou rénaux [O.M.S. (16), J.N.C. (17)].

Il importe, selon le demandeur, de proposer des inhibiteurs de l'ECA qui présentent des valeurs d'IC₅₀ qui soient nettement inférieures à celles des trois peptides de la caséine α_{S2}, cités ci-dessus. Par valeurs nettement inférieures, on peut retenir des valeurs de l'ordre de ou inférieures à 60 µM, sachant toutefois qu'il subsiste une certaine imprécision quant à la valeur obtenue en fonction des conditions opératoires et qu'il convient donc de se reporter aux conditions décrites ci-après pour la détermination de ladite valeur.

Or le demandeur a trouvé par des tests *in vitro* que certains peptides de la caséine α_{S2} présentent une activité inhibitrice sur l'ECA, non mentionnée jusqu'à lors, avec des valeurs de l'ordre de ou inférieures à 60 µM. Il s'agit de cinq peptides qui peuvent être obtenus par hydrolyse trypsique de la caséine α_{S2} à savoir CNα_{S2}-(f25-32), CNα_{S2}-(f92-98), CNα_{S2}-(f174-179), CNα_{S2}-(f174-181), CNα_{S2}-(f182-184) et de deux autres peptides obtenus par synthèse chimique à savoir CNα_{S2}-(f25-30) et CNα_{S2}-(f174-177).

C'est donc l'objet de la présente invention que de revendiquer l'utilisation pour la préparation de médicaments à activité de type anti-hypertensive, utiles pour le traitement ou la prévention de l'hypertension, d'un ou plusieurs peptides, ayant une activité inhibitrice vis-à-vis de l'ECA avec des valeurs d'IC₅₀ de l'ordre de ou inférieures à 60 µM, choisis parmi le groupe de peptides ayant les séquences en acides aminés ci-après :

La présente invention concerne également les compositions pharmaceutiques contenant à titre d'ingrédient actif une quantité efficace d'au moins un desdits peptides en combinaison avec un véhicule pharmaceutiquement acceptable.

L'invention concerne également les produits alimentaires qui contiennent à titre de principe actif au moins un desdits peptides, ou bien de l'hydrolysat trypsique total contenant au moins un desdits peptides ou bien une fraction de cet hydrolysat contenant au moins un desdits peptides en combinaison avec des supports alimentaires, notamment de nature protéique, lipidique ou glucidique. Ces compléments alimentaires peuvent convenir pour supplémenter l'alimentation des personnes sujettes notamment à l'hypertension ou afin de prévenir son apparition.

Dans le groupe de peptides de la présente invention,
le peptide Thr-Val-Tyr, [TVY (SEQ ID NO : 1)], de poids moléculaire 381,4, correspond au peptide 182-184 de la caséine α_{S2},
le peptide Asn-Met-Ala-Ile-Asn-Pro-Ser-Lys, [NMAINPSK (SEQ ID NO : 2)], de poids moléculaire 874,0, correspond au peptide 25-32 de la caséine α_{S2},
le peptide Phe-Ala-Leu-Pro-Gln-Tyr, [FALPQY (SEQ ID NO : 3)], de poids moléculaire 737,9, correspond au peptide 174-179 de la caséine α_{S2},
le peptide Phe-Pro-Gln-Tyr-Leu-Gln-Tyr, [FPQYLQY (SEQ ID NO : 4)], de poids moléculaire 958,1, correspond au peptide 92-98 de la caséine α_{S2},
le peptide Phe-Ala-Leu-Pro-Gln-Tyr-Leu-Lys, [FALPQYLK (SEQ ID NO : 5)], de poids moléculaire 979,2, correspond au peptide 174-181 de la caséine α_{S2},
le peptide Asn-Met-Ala-Ile-Asn-Pro, [NMAINP (SEQ ID NO : 6)] de poids moléculaire 658,8, correspond au peptide 25-30 de la caséine α_{S2},
le peptide Phe-Ala-Leu-Pro, [FALP (SEQ ID NO: 7)], de masse moléculaire 446,6, correspond au peptide 174-177 de la caséine α_{S2}.

Certains de ces peptides peuvent être obtenus à partir de la caséine α_{S2} par hydrolyse enzymatique, de préférence à l'aide de la trypsine. Ils peuvent être ensuite concentrés ou isolés par chromatographie liquide haute performance (CLHP) en phase inverse et autres techniques de chromatographie (gel filtration, échange d'ions, etc...), par centrifugation (sur membrane) et autres techniques de séparation sur membrane (microfiltration, ultrafiltration, etc...).

Ces peptides peuvent aussi être obtenus par synthèse chimique selon les procédés bien connus de l'homme de l'art, tels que ceux décrits par exemple par MERRIFIELD (18).

La caséine entière est obtenue à partir du lait par précipitation acide et neutralisation à l'aide d'un alcali selon des procédés bien connus. Par exemple, on peut utiliser la méthode de NITSCHMANN et LEHMANN (19).

La caséine αₛ₂, utilisée comme produit de départ pour l'obtention de peptides du groupe sélectionné dans le cadre de la présente invention, peut être obtenue par les procédés classiques bien connus de l'homme du métier à partir du lait, de caséines entières, de caséinates et de concentrés de protéines totales du lait, obtenus par exemple selon les procédés décrits par THOMSON (20) et MAUBOIS (21).

Par exemple on peut préparer la caséine α_{S2} en adaptant la méthode décrite par SANOGO *et al*. (22). Cette méthode est une méthode de fractionnement sur DEAE-cellulose utilisant un gradient discontinu de chlorure de calcium comme éluant. Elle permet de fractionner rapidement l'ensemble des caséines. Elle peut être avantageusement mise en oeuvre avec, comme support échangeur d'anions, la DEAE-cellulose DE 23 [commercialisée par Whatman, Maidstone, Grande-Bretagne] qui est une résine sèche. Après cette étape, afin d'éliminer toute trace d'autres protéines, une étape supplémentaire de chromatographie d'interaction hydrophobe en appliquant un gradient décroissant en phosphate de sodium sur une colonne TSKgel phenyl 5PW [TosoHaas, Stuttgart, Allemagne] peut être réalisée.

L'hydrolysat trypsique total de la caséine α_{S2} est obtenu par action de la trypsine sur la caséine α_{S2} par exemple dans les conditions décrites ci-après.

Les premier, deuxième, troisième, quatrième et cinquième peptides [SEQ ID NO : 1, 2, 3, 4, 5] du groupe sélectionné dans le cadre de la présente invention sont purifiés, directement à partir de l'hydrolysat trypsique total, par fractionnement par CLHP en phase inverse à l'aide d'un gradient d'acétonitrile. Les pics peptidique collectés, correspondant à chacun de ces cinq peptides, sont lyophilisés.

Chacun de ces cinq peptides, seul ou en mélange, ou bien une fraction de l'hydrolysat trypsique total contenant au moins l'un de ces cinq peptides ou bien l'hydrolysat trypsique total contenant les cinq peptides peut être utilisé comme principe actif soit dans des compléments alimentaires en combinaison avec des supports alimentaires (par exemple des protéines, des lipides ou des glucides), soit dans des produits alimentaires destinés à une alimentation particulière.

Les médicaments utiles pour le traitement de l'hypertension préparés avec au moins l'un des sept peptides du groupe sélectionné dans le cadre de la présente invention peuvent être administrés par voie orale.

Pour une administration par voie orale, les compositions pharmaceutiques peuvent être sous forme de comprimés, gélules, poudres, granulés ou toute autre forme administrable par voie orale.

L'invention va être maintenant décrite plus en détail par l'exemple ci-après non limitatif :

### A - Préparation de la caséine α_{S2}

Cinq grammes de caséinate d'ammonium sont dissous dans 200 mL de tampon acétate 20 mM, pH 6,6 contenant 3,3 M d'urée, 35 mM d'EDTA et 0,1% de 2-mercaptoéthanol puis on ajoute 20 g de DEAE-cellulose DE 23 équilibrée dans 150 mL du même tampon. Le mélange résultant est agité pendant 15 min à 25°C puis filtré sur un filtre n°41 [Whatman]. Le rétentat est élué avec 2 fois 250 mL de tampon acétate-urée-EDTA sans 2-mercaptoéthanol. Les trois filtrats sont regroupés. Ce premier cycle d'agitation-filtration permet d'éliminer une fraction F0. Les fractions caséiniques suivantes (F1 et F2) sont éluées selon la même procédure en rajoutant au tampon 30 et 70 mM de CaCl₂ respectivement. De l'EDTA est ajouté aux fractions à raison de 15 mM dans la fraction F0, 45 mM dans la fraction F1 et 85 mM dans la fraction F2. Les filtrats F0, F1, F2, dialysés contre de l'eau ultra-pure puis lyophilisés, sont soumis à une électrophorèse en gel de polyacrylamide-urée afin de visualiser le fractionnement. La fraction F1 contient la caséine α_{S2}.

La purification de la caséine α_{S2} est achevée par chromatographie d'interactions hydrophobes sur une colonne TSKgel phenyl 5PW [TosoHaas, Stuttgart, Allemagne] 150 x 21,5 mm. La fraction F1 (1 mg.mL⁻¹) est mise en solution dans un tampon phosphate de sodium 0,48 M, pH 6,4, contenant 2,5 M d'urée et en présence de 0,1% de 2-mercaptoéthanol puis filtrée sur un filtre 0,45 µm PVDF [Pall Corporation, Ann Arbor, Michigan, Etats-Unis]. Vingt milligrammes de solution protéique sont injectés. Un gradient non-linéaire de 0,48 M à 0,037 M en phosphate de sodium pH 6,4 contenant 2,5 M d'urée est appliqué avec un débit de 6,0 mL.min⁻¹ comme suit : de 480 mM à 126 mM (18 min), 126 mM (3 min), de 126 mM à 103 mM (3 min), 103 mM (3 min), de 103 mM à 72 mM (5 min), 72 mM (5 min), de 72 mM à 37 mM (4 min), 37 mM (17 min). La caséine α_{S2} bovine collectée est dialysée, lyophilisée et stockée sous vide à +4°C.

### B - Préparation de l'hydrolysat trypsique de la caséine α_{S2}

La caséine α_{S2} est mise en solution à la concentration de 0,05% (p/v) dans 100 mL de tampon phosphate de sodium 67 mM, pH 8,1 contenant 0,02% d'azoture de sodium. La trypsine (E.C. 3.4.21.4) pancréatique bovine immobilisée sur billes d'agarose et traitée par la TPCK (N-tosyl-L-phénylalanine chlorométhylcétone) [Sigma, Saint-Louis, Missouri, Etats-Unis] est ajoutée, après plusieurs lavage dans le tampon précédent et filtration, à la solution de caséine α_{S2} pour obtenir une concentration de 0,2 unités Nα-benzoyl-L-arginine ethyl ester (BAEE) par ml. L'hydrolyse se déroule à 37°C pendant 24 heures. La réaction est stoppée en diluant deux fois le mélange à l'aide d'acétonitrile 4% contenant 0,2% d'acide trifluoroacétique (TFA), puis en filtrant sur un filtre 0,45 µm PVDF. L'hydrolysat est conservé à -30°C.

### C - Fractionnement de l'hydrolysat par CLHP-phase inverse en gradient d'acétonitrile

L'hydrolysat est fractionné sur colonne C18 XTerra™ [Waters, Milford, Massachussets, Etats-Unis] 250 x 4,6 mm thermostatée à 37°C. 500 µL d'échantillon (0,25 mg.mL⁻¹) sont injectés. Le profil d'élution comporte une phase isocratique de 3 min à 1,6% d'acétonitrile dans l'eau (en présence de 0,1% de TFA) suivie d'un gradient linéaire permettant d'atteindre 40% d'acétonitrile en 87 min au débit d'1 mL.min⁻¹.

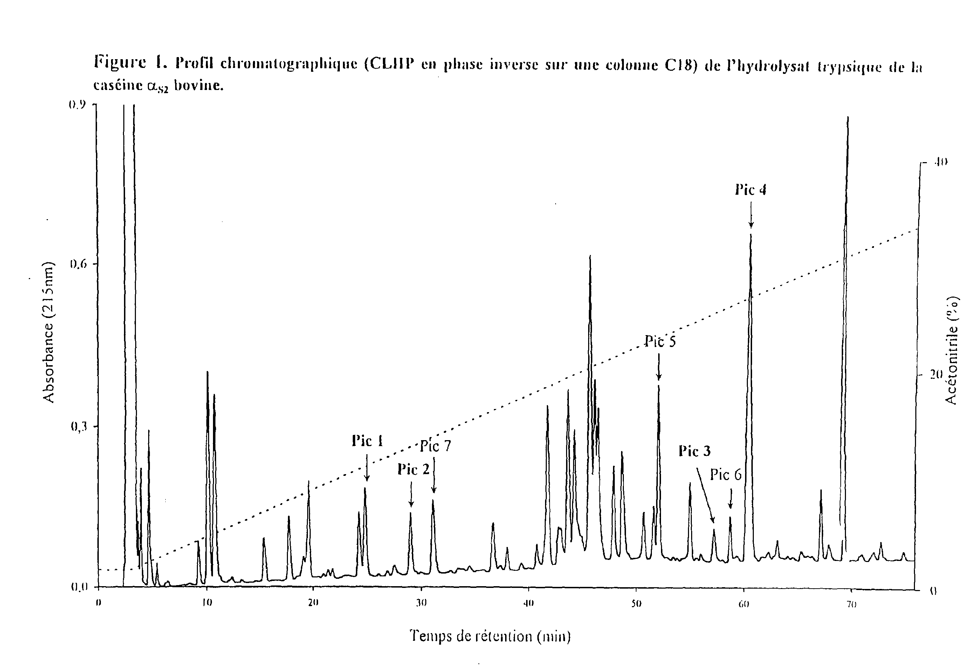

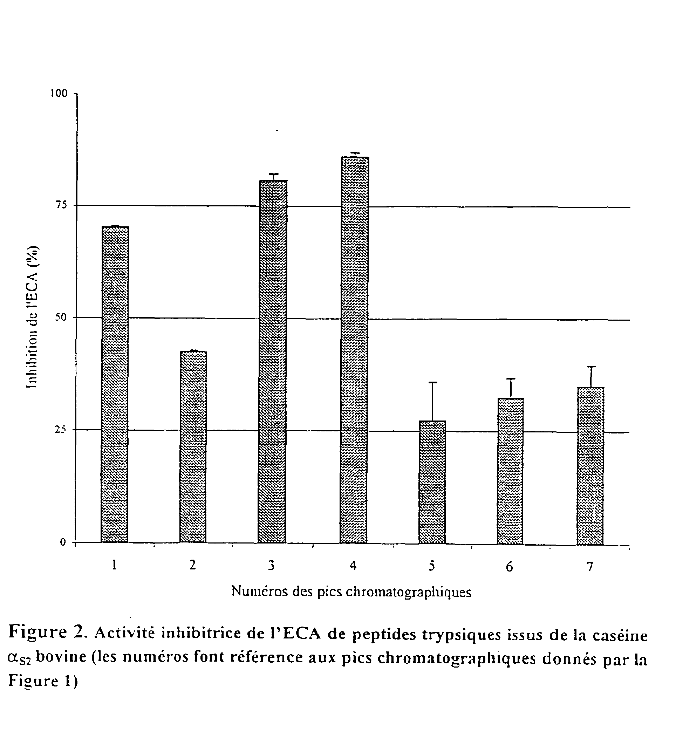

Le profil peptidique est représenté sur la figure 1 où l'absorbance à 215 nm est portée en ordonnée et le temps d'élution en abscisse.

Cinq des sept peptides du groupe sélectionné dans le cadre de la présente invention correspondent aux pics peptidiques référencés de 1 à 4 sur la figure 1. Ces peptides sont collectés et lyophilisés deux fois. Leur identification est réalisée en déterminant leur composition en acides aminés par la méthode à la ninhydrine de HAMILTON (23) ainsi que par spectrométrie de masse couplée à la CLHP, ESI-LC/MS ("electrospray source ionization" ou ionisation electrospray), voire par MS/MS, spectrométrie de masse en tandem.
Le pic 1 collecté à 25 min contient le peptide TVY (SEQ ID NO : 1).
Le pic 2 collecté à 29 min contient le peptide NMAINPSK (SEQ ID NO : 2).
Le pic 3 collecté à 57 min contient le peptide FALPQY (SEQ ID NO : 3).
Le pic 4 collecté à 60 min contient les peptides FPQYLQY (SEQ ID NO : 4) et FALPQYLK (SEQ ID NO : 5).

Les deux autres peptides, à savoir NMAINP (SEQ ID NO : 6) et FALP (SEQ ID NO : 7), peuvent être obtenus par synthèse chimique selon les procédés conventionnels. Il en est d'ailleurs de même pour les cinq peptides obtenus préférentiellement par fractionnement de l'hydrolysat trypsique total de caséine α_{S2}.

### D - Test in vitro des peptides sur l'enzyme de conversion de l'angiotensine 1 (ECA)

Le principe de l'expérience repose sur la mesure de l'activité résiduelle de l'ECA sur un substrat de synthèse, l'Hippuryl-His-Leu-OH, en présence d'un peptide potentiellement inhibiteur [CUSHMAN and CHEUNG (24)]. L'acide hippurique libéré est dosé par CHLP et sa quantité est comparée à un témoin sans inhibiteur.

L'incubation est réalisée dans un tampon CHES 50 mM, pH 8,3, contenant 5 mM d'Hippuryl-His-Leu-OH, 350 mM de NaCI, 3,33 U.L⁻¹ d'ECA et 5% d'éthanol. Le mélange (volume final : 150 µL), après 10 min de préincubation sans l'enzyme, est incubé 60 min à 37°C. La réaction est arrêtée à l'aide de captopril (5 µM), d'EDTA (1 mM) et de TFA (0,067%). L'acide hippurique libéré est quantifié par CLHP en utilisant une colonne C18 Symmetry^{®} [Waters, Milford, Massachussets, Etats-Unis] 150 x 2,1 mm thermostatée à 37°C. Les échantillons sont filtrés sur filtre 0,45 µm PVDF et 40 µL sont injectés. Un gradient d'acétonitrile dans l'eau (en présence de 0,1 % de TFA) est appliqué à un débit de 0,25 mL.min⁻¹. Ce gradient d'élution passe de 13 à 50% d'acétonitrile en 7 min, puis atteint 99% en 0,5 min et est maintenu à cette valeur durant 1,5 min.

La méthode de détermination des IC₅₀ est validée en comparant la valeur trouvée pour le captopril (0,022 µM), un inhibiteur de l'ECA connu, aux valeurs bibliographiques (0,023 µM [CUSHMAN et al. (25)], 0,018 µM [DUNCAN et al. (26)], 0,007 µM [PIHLANTO-LEPPÄLÄ et al. (27)]).

Les quatre pics chromatographiques (1 à 4) collectés à partir de l'hydrolysat trypsique de caséine α_{S2} et correspondant aux cinq peptides du groupe sélectionné dans le cadre de la présente invention sont testés deux fois à une concentration de 50 µM en amines primaires. Les pics chromatographiques numérotés de 5 à 7 sont testés dans les mêmes conditions.

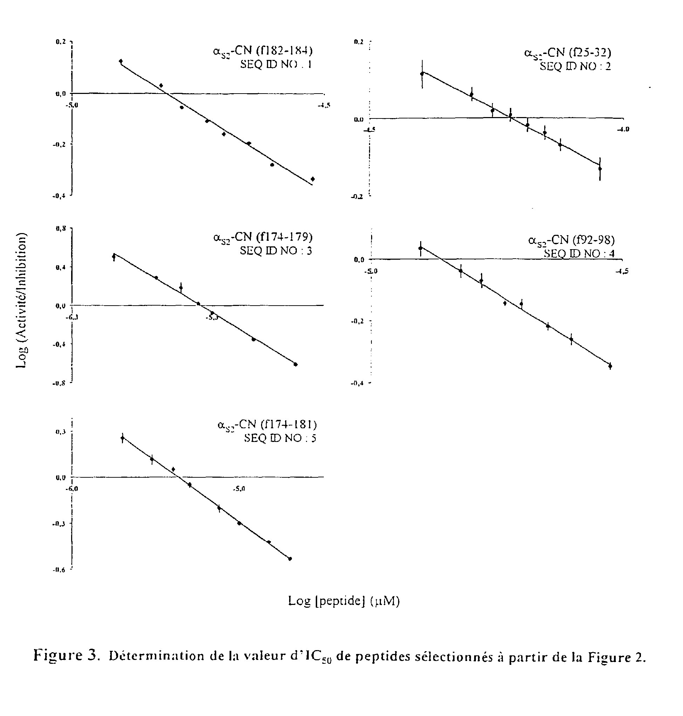

Les résultats obtenus sont présentés sur la figure 2 où le pourcentage d'inhibition est porté en ordonnée et le numéro du pic chromatographique en abscisse. On constate que les pics 1 à 4 contenant les peptides du groupe sélectionné dans le cas de la présente invention inhibent l'ECA à plus de 40%, parmi lesquels le pic 4 contenant les peptides FPQYLQY (SEQ ID NO : 4) et FALPQYLK (SEQ ID NO : 5), le pic 3 contenant le peptide FALPQY (SEQ ID NO : 3) et le pic 1 contenant le peptide TVY (SEQ ID NO : 1) inhibent l'ECA à plus de 70%.

Des peptides de synthèse sont utilisés pour déterminer précisément les IC₅₀ de ces 5 peptides. Les peptides sont testés deux fois dans un premier temps à des concentrations comprises entre 0,1 et 250 à 500 µM pour obtenir une estimation de leur IC₅₀, puis testés en triple sur une gamme de concentrations appropriée.

Les résultats obtenus sont présentés sur les graphes de la figure 3 où le logarithme du rapport activité/inhibition est porté en ordonnée et le logarithme de la concentration en peptide en abscisse. Ceci permet de linéariser la courbe d'inhibition et d'en déduire des valeurs d'IC₅₀ d'après l'équation des droites. Les valeurs d'IC₅₀ sont récapitulées dans le tableau 1.

Elles sont toutes de l'ordre ou inférieures à 60 µM, étant noté que les peptides FALPQY (SEQ ID NO : 3) et FALPQYLK (SEQ ID NO : 5) sont les plus performants avec une valeur d'IC₅₀ de 4,3 µM.

Les sept peptides du groupe sélectionné dans le cadre de la présente invention ont des séquences en acides aminés différentes de celles des peptides inhibiteurs de l'ECA décrites à ce jour [FITZGERALD and MEISEL (28), YAMAMOTO and TAKANO (29), PIHLANTO-LEPPÄLÄ (30), NURMINEN (31), TAKANO (32)] y compris de celles rapportées par MAENO *et al.* (13) obtenues à partir de la caséine α_{S2}: CNα_{S2}-(f198-202), CNα_{S2}-(f190-197) et CNα_{S2}-(f189-193). Comme précisé ci-dessus, deux peptides du groupe sélectionné dans le cadre de la présente invention, obtenus par fractionnement de l'hydrolysat trypsique de la caséine α_{S2} ont une IC₅₀ inférieure à 5 µM et deux autres ont une IC₅₀ inférieure à 20 µM, ce qui les classe parmi les inhibiteurs les plus actifs de l'ECA parmi les peptides naturels obtenus par un processus mono- enzymatique sur des protéines du lait.

En ce qui concerne les deux peptides NMAINP (SEQ ID NO : 6) et FALP (SEQ ID NO : 7), qui ne sont pas obtenus directement par fractionnement de l'hydrolysat trypsique de la caséine α_{S2}, ils sont remarquables d'une part en ce qu'ils possèdent un résidu prolyl à leur extrémité C-terminale, ce qui est commun à certains autres peptides inhibiteurs de l'ECA [MARUYAMA *et al.* (33), KOHMURA *et al.* (34, 35, 36), NAKAMURA *et al.* (37)], et d'autre part en ce que leur séquence en acides aminés est entièrement comprise dans deux autres peptides NMAINPSK (SEQ ID NO : 2) et FALPQY (SEQ ID NO : 3) qui sont obtenus directement par un tel fractionnement. De ce fait, il est envisageable que l'utilisation comme médicament ou complément alimentaire de ces deux derniers peptides (SEQ ID NO 2 et 3) puisse conduire, par rupture de la liaison peptidique adéquate, à la formation *in vivo* des deux premiers peptides (SEQ ID NO : 6 et 7).

Il est à noter que l'utilisation d'au moins un des sept peptides du groupe sélectionné dans le cadre de la présente invention pour la préparation de médicaments, d'aliments ou de compléments alimentaires peut se faire en combinaison avec un ou plusieurs autres peptides, ayant une activité inhibitrice de l'ECA mais ayant une valeur d'IC₅₀ supérieure à 60 µM. Ce serait le cas lors de la mise en oeuvre de l'hydrolysat trypsique total de la caséine α_{S2} ou d'une fraction de celui-ci, contenant au moins un peptide du groupe. Cette combinaison peut s'avérer profitable pour l'activité inhibitrice *in vivo* vis-à-vis de l'ECA.

De préférence cette combinaison ferait intervenir les peptides suivants :
(SEQ ID NO : 8), CNα_{S2}-(f81-91), ALNEINQFYQK, Ala-Leu-Asn-Glu-lle-Asn-Gln-Phe-Tyr-Gln-Lys, pic 5 élué à 52 min,
(SEQ ID NO : 9), CNα_{S2}-(f81-89), ALNEINQFY, Ala-Leu-Asn-Glu-Ile-Asn-Gln-Phe-Tyr, pic 6 élué à 59 min,
(SEQ ID NO : 10), CNα_{S2}-(f206-207), YL, Tyr-Leu, pic 7 élué à 31 min,
qui peuvent aussi être obtenus par fractionnement de l'hydrolysat trypsique de la caséine α_{S2} et qui inhibent l'ECA entre 25 et 35% à une concentration de 50 µM en amines primaires (Figure 2).

### Références bibliographiques

(1) GROSCLAUDE, F., 1988, Le polymorphisme des principales lactoprotéines bovines, INRA Prod. Anim., 1, 5-17.
(2) SWAISGOOD, H. E., 1992, Chemistry of the caseins in P. F. Fox: Advanced dairy chemistry, volume 1, Proteins, Blackie Academic & Professional, London, United Kingdom, 63-109.
(3) GRAPPIN, R. and RIBADEAU-DUMAS, B., 1992, Analytical methods for milk proteins in P. F. Fox: Advanced dairy chemistry, volume 1, Proteins, Blackie Academic & Professional, London, United Kingdom, 1-61.
(4) EIGEL, W. N., BUTLER, J. E., ERNSTROM, C. A., FARRELL, H. M., HARWALKAR, V. R., JENNESS, R. and WHITNEY, R. McL., 1984, Nomenclature of proteins of cow's milk : fifth revision, J. Dairy Sci., 67, 1599-1631.
(5) HOLT, C. and SAWYER, L., 1988, Primary and predicted secondary structures of the caseins in relation to their biological functions, Protein Eng., 2, 251-259.
(6) BRIGNON, G., RIBADEAU-DUMAS, B., MERCIER, J.-C., PELISSIER, J.-P. and DAS, B. C., 1977, Complete amino acid sequence of bovine α_{S2}-casein, FEBS Lett., 76, 274-279.
(7) STEWART, A.F., BONSING, J., BEATTIE, C. W., SHAH, F., WILLIS, I. M. and MACKINLAY, A. G., 1987, Complete nucleotide sequence of bovine α_{S2} and β-casein cDNAs: comparisons with related séquences in other species, Mol. Biol. Evol., 4, 231-241.
(8) CLARE, D. A. and SWAISGOOD, H. E., 2000, Bioactive milk peptides: a prospectus, J. Dairy Sci., 83, 1187-1195.
(9) MEISEL, H., 1997, Biochemical properties of regulatory peptides derived from milk proteins, Biopolymers, 43, 119-128.
(10) ZUCHT, H.-D., RAIDA, M., ADERMANN, K, MÄGERT, H.-J. and FORSSMANN, W.-G., 1995, Casocidin-1: a casein-α_{S2} derived peptide exhibits antibacterial activity, FEBS Lett., 372, 185-188.
(11) RECIO, I. and VISSER, S., 1999, Identification of two distinct antibacterial domains within the séquence of bovine-α_{S2}, Biochim. Biophys. Acta, 1428, 314-326.
(12) CORVOL, P., WILLIAMS, T. A., SOUBRIER, F., 1995, Peptidyl dipeptidase A: angiotensin l-converting enzyme, Methods Enzymol., 248, 243-305.
(13) MAENO, M., YAMAMOTO, N. and TAKANO, T., 1996, Identification of an antihypertensive peptide from casein hydrolysate produced by a proteinase from *Lactobacillus helveticus* CP790, J. Dairy Sci., 79, 1316-1321.
(14) WEBER, M. A., 1999, Interrupting the renin-angiotensin system: the role of angiotensin-converting enzyme inhibitors and angiotensin II receptor antagonists in the treatment of hypertension, 12, 189S-194S.
(15) PIEPHO, R. W., 2000, Overview of the angiotensin-converting-enzyme inhibitors, Am. J. Health-Syst. Pharm., 57, S3-S7.
(16) Guidelines subcommittee, 1999, World Health Organization-International Society of Hypertension. Guidelines for the management of hypertension, J. Hypertens., 17, 151-183.
(17) Joint National Committee, 1997, Détection and treatment of high blood pressure. The sixth report of the joint national committee on prévention and treatment of high blood pressure (JNC VI), Arch. Intern. Med., 157, 2413-2446.
(18) MERRIFIELD, R. B., 1963, Solid phase peptide synthesis I. Synthesis of a tetrapeptide, J. Amer. Chem. Soc., 85, 2149-2154.
(19) NITSCHMANN, H. S. and LEHMANN, W., 1947, Zum problem der labwirkung auf casein, Helv. Chim. Acta, 130, 804.
(20) THOMSON, A. R., 1984, Recent developments in protein recovery and purification, J. Chem. Tech. Biotechnol., 34B, 190-198.
(21) MAUBOIS, J.-L., 1984, Separation, extraction and purification of milk protein components, Lait, 64, 485-495.
(22) SANOGO, T., PAQUET, D., AUBERT, F. and LINDEN, G., 1989. Purification of α_{S1}-casein by fast protein liquid chromatography, J. Dairy Sci., 72, 2242-2246.
(23) HAMILTON, P. B., 1963, Ion exchange chromatography of amino acids. A single column, high resolving, fully automatic procédure, Anal. Chem., 35, 2055-2063.
(24) CUSHMAN, D. W. and CHEUNG, H. S., 1971, Spectrophotometric assay and properties of the angiotensin-converting enzyme of rabbit lung, Biochem. Pharm., 20, 1637-1648.
(25) CUSHMAN, D. W., CHEUNG, H. S., SABO, E. F. and ONDETTI, M. A., 1977, Design of potent compétitive inhibitors of angiotensin-converting enzyme. Carboxyalkanoyl and mercaptoalkanoyl amino acids, Biochemistry, 16, 5484-5491.
(26) DUNCAN, A. C., JÄGER, A. K. and VAN STADEN, J., 1999, Screening of Zulu medicinal plants for angiotensin converting enzyme (ACE) inhibitors, J. Ethnopharm., 68, 63-70.
(27) PIHLANTO-LEPPÂLÂ, A., ROKKA, T. and KORHONEN, H., 1998, Angiotensin I converting enzyme inhibitory peptides derived from bovine milk proteins, Int. Dairy J., 8, 325-331.
(28) FITZGERALD, R. J. and MEISEL, H., 2000, Milk protein-derived inhibitors of angiotensin-l-converting enzyme, British J. Nutr., 84, S33-S37.
(29) YAMAMOTO, N. and TAKANO, T., 1999, Antihypertensive peptides derived from milk proteins, Nahrung, 3, S159-S164.
(30) PIHLANTO-LEPPÄLÄ, A., 2001, Bioactive peptides derived from bovine whey proteins: opioid and ace-inhibitory peptides. Trends Food Sci. Tech., 11, 347-356.
(31) NURMINEN, M.-L., 2000, Milk-derived peptides and blood pressure, Bull. IDF, 353, 11-15.
(32) TAKANO, T., 1998, Milk derived peptides and hypertension-reduction, Int. Dairy J., 8, 375-381.
(33) MARUYAMA, S., NAKAGOMI, K., TOMIZUKA, N. and SUZUKI, H., 1985, Angiotensin 1-converting enzyme inhibitor derived from an enzymatic hydrolysate of casein. II. Isolation and bradykinin-potentiating activity on the utérus and the ileum of rats, Agric. Biol. Chem., 49, 1404-1409.
(34) KOHMURA, M., NIO, N., KUBO, K. MINOSHIMA, Y., MUNEKATA, E. and ARIYOSHI, Y., 1989, Inhibition of angiotensin-converting enzyme by synthetic peptides of human β-casein, Agric. Biol. Chem., 53, 2107-2114.
(35) KOHMURA, M., NIO, N. and ARIYOSHI, Y., 1990a, Inhibition of angiotensin-converting enzyme by synthetic fragments of human K-casein, Agric. Biol. Chem., 54, 835-836.
(36) KOHMURA, M., NIO, N. and ARIYOSHI, Y., 1990b, Inhibition of angiotensin-converting enzyme by synthetic peptide fragments of various β-casein, Agric. Biol. Chem., 54, 1101-1102.
(37) NAKAMURA, Y., YAMAMOTO, N., SAKAI, K. and TAKANO, T., 1995, Antihypertensive effect of sour milk and peptides isolated from it that are inhibitors to angiotensin I converting enzyme, J. Dairy Sci., 78, 1253.

**Tableau 1**

| Inhibiteur | N°^{a} | Séquence | ID NO^{b} | Inhibition (%)^{c} | IC₅₀ (µM) |
|---|---|---|---|---|---|
| Captopril | | | | > 99.5 | 0.022 |
| CNα_{S2}-(f 182-184) | 1 | TVY | 1 | 70.2 | 15 |
| CNα_{S2}-(f25-32) | 2 | NMAINPSK | 2 | 42.5 | 60 |
| CNα_{S2}-(f 174-179) | 3 | FALPQY | 3 | 82.7 | 4.3 |
| CNα_{S2}-(f 92-98) | 4 | FPQYLQY | 4 | 86.0^{d} | 14 |
| CNα_{S2}-(f 174-181) | 4 | FALPQYLK | 5 | 86.0^{d} | 4.3 |
| CNα_{S2}-(f 81-91) | 5 | ALNEINQFYQK | 8 | 27.2 | 264 |
| CNα_{S2}-(f 81-89) | 6 | ALNEINQFY | 9 | 32.2 | 219 |
| CNα_{S2}-(f 206-207) | 7 | YL | 10 | 34.8 | nd |

| | | | | | |
|---|---|---|---|---|---|
| ^{a} numéro du pic en CLHP sur la figure 1 ; ^{b} numéro d'identification de la séquence du peptide ; ^{c} déterminé avec une concentration en amines primaires ou en captopril égale à 50 µM : ^{d} CNα_{S2}-(f92-98) et CNα_{S2}-(f174-181) étaient mélangés dans le pic n°4 : nd. non déterminée. | | | | | |

## Revendications

1. Utilisation pour la préparation de médicaments à activité de type antihypertensive, utiles pour le traitement ou la prévention de l'hypertension d'un ou plusieurs peptides, ayant une activité inhibitrice vis-à-vis de l'ECA avec des valeurs d'IC₅₀ de l'ordre de ou inférieures à 60 µM, choisis parmi le groupe de peptides ayant les séquences en acides aminés ci-après :
Thr-Val-Tyr ( SEQ ID NO : 1 )
Asn-Met-Ala-Ile-Asn-Pro-Ser-Lys ( SEQ ID NO : 2 )
Phe-Ala-Leu-Pro-Gln-Tyr ( SEQ ID NO : 3 )
Phe-Pro-Gln-Tyr-Leu-Gln-Tyr ( SEQ ID NO : 4 )
Phe-Ala-Leu-Pro-Gln-Tyr-Leu-Lys ( SEQ ID NO : 5 )
Asn-Met-Ala-Ile-Asn-Pro ( SEQ ID NO : 6 )
Phe-Ala-Leu-Pro ( SEQ ID NO : 7 )

2. Composition pharmaceutique contenant à titre de principe actif une quantité efficace d'un ou plusieurs peptides, ayant une activité inhibitrice vis-à-vis de l'ECA avec des valeurs d'IC₅₀ de l'ordre ou inférieures à 60 µM, choisis parmi le groupe de peptides ayant les séquences en acides aminés ci-après :
Thr-Val-Tyr (SEO ID NO : 1 )
Asn-Met-Ala-Ile-Asn-Pro-Ser-Lys ( SEQ ID NO : 2 )
Phe-Ala-Leu-Pro-Gln-Tyr ( SEQ ID NO : 3 )
Phe-Pro-Gln-Tyr-Leu-Gln-Tyr ( SEQ ID NO : 4)
Phe-Ala-Leu-Pro-Gln-Tyr-Leu-Lys ( SEQ ID NO : 5 )
Asn-Met-Ala-Ile-Asn-Pro ( SEQ ID NO : 6 )
Phe-Ala-Leu-Pro ( SEQ ID NO : 7 )
en combinaison avec un véhicule pharmaceutiquement acceptable.

3. Produit alimentaire, notamment utile pour supplémenter l'alimentation des personnes sujettes à l'hypertension ou désireuses de prévenir son apparition, contenant une quantité efficace d'un ou plusieurs peptides, ayant une activité inhibitrice vis-à-vis de l'ECA avec des valeurs d'IC₅₀ de l'ordre ou inférieures à 60 µM, choisis parmi le groupe de peptides ayant les séquences en acides aminés ci-après
Thr-Val-Tyr ( SEQ ID NO : 1)
Asn-Met-Ala-Ile-Asn-Pro-Ser-Lys ( SEQ ID NO : 2)
Phe-Ala-Leu-Pro-Gln-Tyr ( SEQ ID NO : 3 )
Phe-Pro-Gln-Tyr-Leu-Gln-Tyr ( SEQ ID NO : 4)
Phe-Ala-Leu-Pro-Gln-Tyr-Leu-Lys ( SEQ ID NO : 5)
Asn-Met-Ala-Ile-Asn-Pro ( SEQ ID NO : 6)
Phe-Ala-Leu-Pro ( SEQ ID NO : 7 )
en combinaison avec des supports alimentaires, notamment de nature protéique, lipidique ou glucidique.

4. Produit alimentaire selon la revendication 3 **caractérisé en ce qu'**il comprend une fraction de l'hydrolysat trypsique de la caséine α_{S2} contenant au moins l'un des peptides ayant les séquences en acides aminés ci-après:
Thr-Val-Tyr ( SEQ ID NO : 1 )
Asn-Met-Ala-Ile-Asn-Pro-Ser-Lys ( SEQ ID NO : 2 )
Phe-Ala-Leu-Pro-Gln-Tyr ( SEQ ID NO : 3 )
Phe-Pro-Gln-Tyr-Leu-Gln-Tyr ( SEQ ID NO : 4)
Phe-Ala-Leu-Pro-Gln-Tyr-Leu-Lys ( SEQ ID NO : 5)

5. Produit alimentaire selon la revendication 3 **caractérisé en ce qu'**il comprend l'hydrolysat trypsique total de la caséine α_{S2} contenant les cinq peptides ayant les séquences en acides aminés ci-après :
Thr-Val-Tyr (SEQ ID NO : 1 )
Asn-Met-Ala-Ile-Asn-Pro-Ser-Lys ( SEQ ID NO : 2 )
Phe-Ala-Leu-Pro-Gln-Tyr ( SEQ ID NO : 3 )
Phe-Pro-Glu-Tyr-Leu-Gln-Tyr ( SEQ ID NO : 4)
Phe-Ala-Leu-Pro-Gln-Tyr-Leu-Lys ( SEQ ID NO : 5 )

6. Produit alimentaire selon l'une des revendications 3 à 5 **caractérisé en ce qu'**il comprend en combinaison au moins l'un des peptides ayant les séquences en acides aminés ci-après :
Ala-Leu-Asn-Glu-Ile-Asn-Gln-Phe-Tyr-Gln-Lys ( SEQ ID NO : 8 )
Ala-Leu-Asn-Glu-Ile-Asn-Gln-Phe-Tyr ( SEQ ID NO : 9 )
Tyr-Leu ( SEQ ID NO : 10 ).

## Claims

1. The use for the preparation of medicines having activity of the antihypertensive type, useful for treating or preventing hypertension, of one or more peptides having inhibiting activity on ACE with IC₅₀ values of the order of or less than 60 µM, selected from the group of peptides having the following amino acid sequences:
Thr-Val-Tyr (SEQ ID No.: 1)
Asn-Met-Ala-Ile-Asn-Pro-Ser-Lys (SEQ ID No.: 2)
Phe-Ala-Leu-Pro-Gln-Tyr (SEQ ID No.: 3)
Phe-Pro-Gln-Tyr-Leu-Gln-Tyr (SEQ ID No.: 4)
Phe-Ala-Leu-Pro-Gln-Tyr-Leu-Lys (SEQ ID No.: 5)
Asn-Met-Ala-Ile-Asn-Pro (SEQ ID No.: 6)
Phe-Ala-Leu-Pro (SEQ ID No.: 7).

2. A pharmaceutical composition containing as its active principle an effective quantity of one of more peptides having inhibiting activity on ACE, with IC₅₀ values of the order of or less than 60 µM, selected from the group of peptides having the following amino acid sequences:
Thr-Val-Tyr (SEQ ID No.: 1)
Asn-Met-Ala-Ile-Asn-Pro-Ser-Lys (SEQ ID No.: 2)
Phe-Ala-Leu-Pro-Gln-Tyr (SEQ ID No.: 3)
Phe-Pro-Gln-Tyr-Leu-Gln-Tyr (SEQ ID No.: 4)
Phe-Ala-Leu-Pro-Gln-Tyr-Leu-Lys (SEQ ID No.: 5)
Asn-Met-Ala-Ile-Asn-Pro (SEQ ID No.: 6)
Phe-Ala-Leu-Pro (SEQ ID No.: 7)
in combination with a pharmaceutically acceptable vehicle.

3. A food product, in particular one that is useful for supplementing the diet of people subject to hypertension or desiring to prevent the appearance thereof, the food product containing an effective quantity of one or more peptides having inhibiting activity on ACE, with IC₅₀ values of the order of or less than 60 µM, selected from the group of peptides having the following amino acid sequences:
Thr-Val-Tyr (SEQ ID No.: 1)
Asn-Met-Ala-Ile-Asn-Pro-Ser-Lys (SEQ ID No.: 2)
Phe-Ala-Leu-Pro-Gln-Tyr (SEQ ID No.: 3)
Phe-Pro-Gln-Tyr-Leu-Gln-Tyr (SEQ ID No.: 4)
Phe-Ala-Leu-Pro-Gln-Tyr-Leu-Lys (SEQ ID No.: 5)
Asn-Met-Ala-Ile-Asn-Pro (SEQ ID No.: 6)
Phe-Ala-Leu-Pro (SEQ ID No.: 7)
in combination with food supports, in particular proteins, lipids, or carbohydrates.

4. A food product according to claim 3, **characterized in that** it includes a fraction of trypsic hydrolysate of α_{S2} casein containing at least one of the peptides having the following amino acid sequences:
Thr-Val-Tyr (SEQ ID No.: 1)
Asn-Met-Ala-Ile-Asn-Pro-Ser-Lys (SEQ ID No.: 2)
Phe-Ala-Leu-Pro-Gln-Tyr (SEQ ID No.: 3)
Phe-Pro-Gln-Tyr-Leu-Gln-Tyr (SEQ ID No.: 4)
Phe-Ala-Leu-Pro-Gln-Tyr-Leu-Lys (SEQ ID No.: 5).

5. A food product according to claim 3, **characterized in that** it includes the total trypsic hydrolysate of α_{S2} casein, containing the five peptides having the following amino acid sequences:
Thr-Val-Tyr (SEQ ID No.: 1)
Asn-Met-Ala-Ile-Asn-Pro-Ser-Lys (SEQ ID No.: 2)
Phe-Ala-Leu-Pro-Gln-Tyr (SEQ ID No.: 3)
Phe-Pro-Gln-Tyr-Leu-Gln-Tyr (SEQ ID No.: 4)
Phe-Ala-Leu-Pro-Gln-Tyr-Leu-Lys (SEQ ID No.: 5).

6. A food product according to any one of claims 3 to 5, **characterized in that** it includes in combination at least one of the peptides having the following amino acid sequences:
Ala-Leu-Asn-Glu-Ile-Asn-Gln-Phe-Tyr-Gln-Lys (SEQ ID No.: 8)
Ala-Leu-Asn-Glu-Ile-Asn-Gln-Phe-Tyr (SEQ ID No.: 9)
Tyr-Leu (SEQ ID No.: 10).

## Patentansprüche

1. Verwendung für die Herstellung von Medikamenten mit blutdrucksenkender Wirkung, die für die Behandlung oder die Vorbeugung von Bluthochdruck nützlich sind, aus einem oder mehreren Peptiden, die eine ACE-hemmende Wirkung aufweisen, mit IC₅₀-Werten von 60 µM oder darunter, ausgewählt aus der Gruppe von Peptiden, die die folgenden Aminosäuresequenzen aufweist:
Thr-Val-Tyr (SEQ-ID NR.: 1),
Asn-Met-Ala-Ile-Asn-Pro-Ser-Lys (SEQ-ID NR.: 2),
Phe-Ala-Leu-Pro-Gln-Tyr (SEQ-ID NR.: 3),
Phe-Pro-Gln-Tyr-Leu-Gln-Tyr (SEQ-ID NR.: 4),
Phe-Ala-Leu-Pro-Gln-Tyr-Leu-Lys (SEQ-ID NR.: 5),
Asn-Met-Ala-Ile-Asn-Pro (SEQ-ID NR.: 6),
Phe-Ala-Leu-Pro (SEQ-ID NR.: 7).

2. Pharmazeutische Zusammensetzung, die als Wirkstoff eine wirksame Menge eines oder mehrerer Peptide enthält, die eine ACE-hemmende Wirkung aufweisen, mit IC₅₀-Werten von 60 µM oder darunter, ausgewählt aus der Gruppe von Peptiden, die die folgenden Aminosäuresequenzen aufweisen:
Thr-Val-Tyr (SEQ-ID NR.: 1)
Asn-Met-Ala-Ile-Asn-Pro-Ser-Lys (SEQ-ID NR.: 2)
Phe-Ala-Leu-Pro-Gln-Tyr, (SEQ-ID NR.: 3),
Phe-Pro-Gln-Tyr-Leu-Gln-Tyr, (SEQ-ID NR.: 4)
Phe-Ala-Leu-Pro-Gln-Tyr-Leu-Lys, (SEQ-ID NR.: 5)
Asn-Met-Ala-Ile-Asn-Pro, (SEQ-ID NR.: 6)
Phe-Ala-Leu-Pro, (SEQ-ID NR.: 7)
in Kombination mit einem pharmazeutisch akzeptablen Hilfsmittel.

3. Nahrungsmittel, das insbesondere nützlich ist, um die Ernährung von Personen zu ergänzen, die an Bluthochdruck leiden oder die dessen Auftreten vorbeugen möchten, das eine wirksame Menge eines oder mehrerer Peptide enthält, die eine ACE-hemmende Wirkung aufweisen, mit IC₅₀-Werten von 60 µM oder darunter, ausgewählt aus der Gruppe von Peptiden, die die folgenden Aminosäuresequenzen aufweisen:
Thr-Val-Tyr (SEQ-ID NR.: 1),
Asn-Met-Ala-Ile-Asn-Pro-Ser-Lys (SEQ-ID NR.: 2),
Phe-Ala-Leu-Pro-Gln-Tyr (SEQ-ID NR.: 3),
Phe-Pro-Gln-Tyr-Leu-Gln-Tyr (SEQ-ID NR.: 4),
Phe-Ala-Leu-Pro-Gln-Tyr-Leu-Lys (SEQ-ID NR.: 5),
Asn-Met-Ala-Ile-Asn-Pro (SEQ-ID NR.: 6),
Phe-Ala-Leu-Pro (SEQ-ID NR.: 7),
in Kombination insbesondere mit eiweißartigen, lipidartigen oder kohlenhydratartigen Nahrungsmittelträgern.

4. Nahrungsmittel nach Anspruch 3, **dadurch gekennzeichnet, daß** es eine Fraktion des tryptischem Hydrolysats des α_{S2}-Caseins umfaßt, die mindestens eines der Peptide enthält, die die folgenden Aminosäuresequenzen aufweisen:
Thr-Val-Tyr (SEQ-ID NR.: 1),
Asn-Met-Ala-Ile-Asn-Pro-Ser-Lys (SEQ-ID NR.: 2),
Phe-Ala-Leu-Pro-Gln-Tyr (SEQ-ID NR.: 3),
Phe-Pro-Gln-Tyr-Leu-Gln-Tyr (SEQ-ID NR.: 4),
Phe-Ala-Leu-Pro-Gln-Tyr-Leu-Lys (SEQ-ID NR.: 5).

5. Nahrungsmittel nach Anspruch 3, **dadurch gekennzeichnet, daß** es das gesamte tryptische Hydrolysat des α_{S2}-Caseins umfaßt, das die fünf Peptide enthält, die die folgenden Aminosäuresequenzen aufweisen:
Thr-Val-Tyr (SEQ-ID NR.: 1),
Asn-Met-Ala-Ile-Asn-Pro-Ser-Lys (SEQ-ID NR.: 2),
Phe-Ala-Leu-Pro-Gln-Tyr (SEQ-ID NR.: 3),
Phe-Pro-Gln-Tyr-Leu-Gln-Tyr (SEQ-ID NR.: 4),
Phe-Ala-Leu-Pro-Gln-Tyr-Leu-Lys (SEQ-ID NR.: 5).

6. Nahrungsmittel nach einem der Ansprüche 3 bis 5, **dadurch gekennzeichnet, daß** es in Kombination wenigstens eines der Peptide umfaßt, die die folgenden Aminosäuresequenzen aufweisen:
Ala-Leu-Asn-Glu-Ile-Asn-Gln-Phe-Tyr-Gln-Lys (SEQ-ID NR.: 8),
Ala-Leu-Asn-Glu-Ile-Asn-Gln-Phe-Tyr (SEQ-ID Nr. 9),
Tyr-Leu (SEQ ID Nr.: 10).


### LISTE DE SEQUENCES

<110> INGREDIA
<120> Utilisation d'au moins un peptide de la caséine alpha (s2) à activité inhibitrice de l'enzyme de conversion de l'angiotensine I pour la préparation de médicaments, d'aliments et de compléments alimentaires
<130> 1H9O487O/OOO4FRO
<160> 10
<170> PatentIn Ver. 2.1
<210> 1
   <211> 3
   <212> PRT
   <213> caséine alpha (s2)
<400> 1
   Thr Val Tyr
    1
<210> 2
   <211> 8
   <212> PRT
   <213> caséine alpha (s2)
<400> 2
   Asn Met Ala Ile Asn Pro Ser Lys
    1               5
<210> 3
   <211> 6
   <212> PRT
   <213> caséine alpha (s2)
<400> 3
   Phe Ala Leu Pro Gln Tyr
    1               5
<210> 4
   <211> 7
   <212> PRT
   <213> caséine alpha (s2)
<400> 4
   Phe Pro Gln Tyr Leu Gln Tyr
    1               5
<210> 5
   <211> 8
   <212> PRT
   <213> caséine alpha (s2)
<400> 5
   Phe Ala Leu Pro Gln Tyr Leu Lys
   1               5
<210> 6
   <211> 6
   <212> PRT
   <213> caséine alpha (s2)
<400> 6
   Asn Met Ala Ile Asn Pro
   1               5
<210> 7
   <211> 4
   <212> PRT
   <213> caséine alpha (s2)
<400> 7
   Phe Ala Leu Pro
   1
<210> 8
   <211> 11
   <212> PRT
   <213> caséine alpha (s2)
<400> 8
   Ala Leu Asn Glu Ile Asn Gln Phe Tyr Gln Lys
   1               5                   10
<210> 9
   <211> 9
   <212> PRT
   <213> caséine alpha (s2)
<400> 9
   Ala Leu Asn Glu Ile Asn Gln Phe Tyr
   1               5
<210> 10
   <211> 2
   <212> PRT
   <213> caséine alpha (s2)
<400> 10
   Tyr Leu
    1

### LISTE DE SEQUENCES

<110> INGREDIA
<120> Utilisation d'au moins un peptide de la caséine alpha (s2) à activité inhibitrice de l'enzyme de conversion de l'angiotensine I pour la préparation de médicaments, d'aliments et de compléments alimentaires
<130> 1H9O487O/OOO4FRO
<160> 10
<170> PatentIn Ver. 2.1
<210> 1
   <211> 3
   <212> PRT
   <213> caséine alpha (s2)
<400> 1
   Thr Val Tyr
    1
<210> 2
   <211> 8
   <212> PRT
   <213> caséine alpha (s2)
<400> 2
   Asn Met Ala Ile Asn Pro Ser Lys
    1               5
<210> 3
   <211> 6
   <212> PRT
   <213> caséine alpha (s2)
<400> 3
   Phe Ala Leu Pro Gln Tyr
    1               5
<210> 4
   <211> 7
   <212> PRT
   <213> caséine alpha (s2)
<400> 4
   Phe Pro Gln Tyr Leu Gln Tyr
    1               5
<210> 5
   <211> 8
   <212> PRT
   <213> caséine alpha (s2)
<400> 5
   Phe Ala Leu Pro Gln Tyr Leu Lys
    1               5
<210> 6
   <211> 6
   <212> PRT
   <213> caséine alpha (s2)
<400> 6
   Asn Met Ala Ile Asn Pro
    1               5
<210> 7
   <211> 4
   <212> PRT
   <213> caséine alpha (s2)
<400> 7
   Phe Ala Leu Pro
    1
<210> 8
   <211> 11
   <212> PRT
   <213> caséine alpha (s2)
<400> 8
   Ala Leu Asn Glu Ile Asn Gln Phe Tyr Gln Lys
    1               5                  10
<210> 9
   <211> 9
   <212> PRT
   <213> caséine alpha (s2)
<400> 9
   Ala Leu Asn Glu Ile Asn Gln Phe Tyr
   1 5
<210> 10
   <211> 2
   <212> PRT
   <213> caséine alpha (s2)
<400> 10
   Tyr Leu
   1